# EUROPÄISCHE PATENTSCHRIFT

(11) **EP 4 017 305 B1**
(45) Veröffentlichungstag und Bekanntmachung des Hinweises auf die Patenterteilung: **02.10.2024**
(21) Anmeldenummer: 20760410.9
(22) Anmeldetag: 18.08.2020
(51) Int. Cl.: A41D 27/13, A61L 15/16, A61L 15/42, A61L 15/52, A61L 15/60

(54) **HYGIENEPFLASTER ZUM ABSORBIEREN VON SCHWEISS**
HYGIENE PATCH FOR ABSORBING SWEAT
TIMBRE HYGIENIQUE POUR ABSORBER LA SUEUR

(30) Priorität: 19.08.2019 DE 102019122253
(43) Veröffentlichungstag der Anmeldung: 29.06.2022
(73) Patentinhaber: Hanse-Lopack Riskau GmbH, 29451 Dannenberg/Elbe (DE)
(72) Erfinder: EGERMANN, Hertha Christine, 21365 Adendorf (DE); MÜLLER-HAUSSCHILDT, Holger, 22763 Hamburg (DE)
(74) Vertreter: Andresen, Heiko
(86) Internationale Anmeldenummer: PCT/EP2020/073047
(87) Internationale Veröffentlichungsnummer: WO 2021/032704

(56) Entgegenhaltungen:
- EP-A1- 3 323 398
- WO-A1-02/46510
- WO-A1-2013/152809
- WO-A1-2018/082881
- GB-A- 2 559 803
- US-A- 5 876 388
- US-B1- 6 269 820

## Beschreibung

### Gebiet der Erfindung

Die vorliegende Erfindung liegt auf dem Gebiet der Körperpflegemittel bzw. kosmetischer Artikel zur Absorption von Schweiß und Vermeidung von Körpergeruch. Insbesondere betrifft die Erfindung selbstklebende Hygienepflaster mit flexibler Trägerschicht und Saugauflage zur Aufbringung auf der Haut in den Achselhöhlen.

### Hintergrund der Erfindung

Die menschliche Haut produziert Schweiß als Teil der thermoregulatorischen Funktion des Körpers und als Reaktion auf emotionalen oder körperlichen Stress. Wie viel Schweiß Menschen produzieren, ist höchst individuell. Eine ausgeprägte Schweißproduktion (Transpiration) wird im Alltag oft als Belastung empfunden, insbesondere wenn der Schweiß zu feuchten Stellen auf der Kleidung (sogenannten Schweißflecken) und zur Bildung von Körpergeruch führt. In der Regel ist dabei der Körperbereich der Achselhöhlen besonders betroffen.

Schweiß ist zunächst nahezu geruchlos und beginnt erst zu riechen, wenn er durch Bakterien auf der Hautoberfläche in unangenehm riechende Substanzen zerlegt wird. Oft werden Deodorants z. B. in Sprayform, als Deoroller oder -stifte, Puder, Gele oder Cremes angewendet, um Schweiß- bzw. Achselgerüche zu überdecken. Alternativ oder zusätzlich werden schweißhemmende Mittel (sogenannte Antitranspirante) verwendet, welche die Aktivität der Schweißdrüsen reduzieren und somit den Körpergeruch vermindern können. Solche Mittel können zwar übergangsweise effektiv sein, verhindern aber selten den Austritt von Schweiß vollständig. Daneben können Deodorants und/oder Antitranspirante zu Verfärbungen oder Schäden an der Kleidung des Benutzers führen. Ein weiteres Problem ist, dass diese Präparate gerade bei empfindlicher Haut Reizungen bzw. allergische Reaktionen hervorrufen können. Auch eine toxische Wirkung von Antitranspirant-Wirkstoffen ist nicht ausgeschlossen.

Eine Möglichkeit, die Kleidung vor den Auswirkungen der Schweißbildung zu schützen, sind absorbierende Polster, die im Achselbereich an der Kleidung angebracht werden. Während diese Polster das Kleidungsstück vor Verfärbung und Flecken schützen können, wird die Geruchsbildung kaum verhindert, da geruchsverursachende Bakterien weiterhin Zugang zu den organischen Verbindungen im Schweiß haben.

Eine weitere Schutzmöglichkeit stellen Hygienepflaster dar, welche in den Achselhöhlen direkt auf die Haut des Benutzers aufgebracht werden, um Schweiß aufzunehmen und schlechte Gerüche zu regulieren.

Aus WO 2018/082881 A1 ist ein solches Pflaster zum Absorbieren von Schweiß von der menschlichen Haut bekannt. Das Pflaster umfasst eine Trägerfolie, eine absorbierende Hydrogelschicht und eine permeable Innenmembran, die zwischen der Hydrogelschicht und der Haut gehalten wird.

WO 2008/154546 A2 offenbart ein weiteres Hygienepflaster mit einer an Haut haftenden Klebstoffschicht, einer flexiblen Rückenschicht und einem fluidabsorbierenden Polster, das einen Abschnitt der Klebstoffschicht überlagert.

US 6,269,820 offenbart eine Wundauflage zur Kontrolle von Leckagen aus chirurgischen Einschnitten. GB 2559803 A offenbart ein Transpirationspflaster mit einer Absorptionsauflage, die ein Fasergemisch aus Viskose und superabsorbierenden Fasern enthält. Weitere saugfähige Strukturen sind in WO 2013/122809 A1, EP 3323398 A1 und US 5,876,388 beschrieben.

Schließlich ist aus US 2006/0251609 ein Pflaster zur Anwendung auf der Haut bekannt, das eine transparente Rückschicht mit einer haftenden Oberfläche, einen Klebstoff, der auf der haftenden Oberfläche aufgebracht ist, eine Ablöseschicht, die lösbar mit dem Klebstoff verbunden ist und ein Mittel zur Reduzierung des Geruchs umfasst.

Nachteilig an den bisher bekannten Hygienepflastern ist, dass sie oft sperrig sind und sich leicht von der Haut lösen können, insbesondere wenn der Benutzer stark schwitzt. Dies kann unter anderem zu einem geringen Tragekomfort führen und die Schutzwirkung des Pflasters gegen Schweißgeruch und -flecken beeinträchtigen.

Es ist daher eine Aufgabe der vorliegenden Erfindung, ein verbessertes Hygienepflaster bereitzustellen, welches die vorgenannten Probleme zumindest teilweise oder vollständig löst.

Diese Aufgabe wird durch die Verwendung eines Hygienepflasters mit den Merkmalen von Anspruch 1 gelöst. Bevorzugte und vorteilhafte Ausgestaltungen der Erfindung ergeben sich aus den weiteren Ansprüchen und der nachstehenden Beschreibung.

### Beschreibung der Erfindung

Es wird ein Hygienepflaster zur Aufnahme von Schweiß menschlicher Haut angegeben, das zur Anwendung bzw. Aufbringung in den Achselhöhlen eines Benutzers ausgebildet ist.

Das Hygienepflaster weist eine Trägerschicht mit einer an der menschlichen Haut fixierbaren Haftoberfläche auf. Die Trägerschicht ist flexibel, d. h. biegsam bzw. elastisch, sodass sich das Hygienepflaster bei der Anwendung an die Körperkonturen anpassen und Bewegungen des Körpers mitmachen kann, ohne die Bewegungsfreiheit wesentlich einzuschränken. Dies verstärkt und schützt das Hygienepflaster beim Tragen gegen Beschädigungen bzw. Verschleiß und erhöht gleichzeitig den Tragekomfort.

Eine geeignete Trägerschicht kann z. B. einen Polymerfilm oder ein Polymervlies aus Polyethylen, Polypropylen, Polyurethan, Polystyrol, Polyvinylchlorid, Polyester, Polyamid und/oder Silikon umfassen oder daraus bestehen. Geeignete Schichtdicken der Trägerschicht liegen im Bereich von etwa 10 Mikrometern bis etwa 500 Mikrometern, vorzugsweise in einem Bereich von etwa 20 Mikrometern bis etwa 300 Mikrometern, besonders bevorzugt in einem Bereich von etwa 25 Mikrometern bis etwa 100 Mikrometern. In einer bevorzugten Ausführung wird die Trägerschicht durch ein querelastisches Polymervlies, z. B. ein Polyestervlies, gebildet.

Die Haftoberfläche umfasst vorzugsweise eine Beschichtung der Innenoberfläche der Trägerschicht mit einem Haftklebstoff, d. h. einem Klebstoff, der unter leichtem Druck eine gute Haftung (Adhäsion) zur menschlichen Haut ausbildet, nachfolgend auch als "Klebstoffschicht" bezeichnet. So, wie der Begriff hier verwendet wird, ist unter der "Innenoberfläche" diejenige Oberfläche der Trägerschicht zu verstehen, welche bei Verwendung des Hygienepflasters zur Haut gewandt ist. Als Haftklebstoffe sind beispielsweise Klebstoffe auf Kautschukbasis wie z. B. Silikonkautschuk, Nitril- bzw. Acrylnitrilkautschuk, Polyisobutylenkautschuk, Chloroprenkautschuk oder Zinkoxid-Kautschuk, Acrylkleber, Silikonkleber oder Polyurethankleber geeignet. Eine weitere Möglichkeit sind Haftkleber, welche ein Styrol-Isopren-Styrol-Polymer, ein Styrol-Olefin-Styrol-Polymer, Polyisobutylen, ein Styrol-Butadien-Styrol-Polymer, Polyisopren oder Polybutadien enthalten. Auch beliebige Kombinationen der vorgenannten Klebstoffe sind möglich. Vorzugsweise handelt es sich bei dem Haftklebstoff um einen hydrophilen Polyacrylatklebstoff bzw. eine selbstklebende Polyacrylatklebemasse, insbesondere um eine wässrige Emulsion eines Acrylesters wie z. B. 2-Ethylhexylacrylat und/oder Ethylacrylat oder eine selbstklebende Zinkoxid-Kautschuk-Klebemasse, mit welcher die Trägerschicht beschichtet ist. Diese Klebstoffe gewährleisten eine zuverlässige Anhaftung des Hygienepflasters an der menschlichen Haut und lassen sich nach Gebrauch einfach und rückstandsfrei wieder entfernen.

Die Beschichtung mit dem Haftklebstoff ist in der Regel dünner als etwa 1 Millimeter. In einer bevorzugten Ausführungsform beträgt die Dicke der Klebstoffschicht etwa 10 Mikrometer bis etwa 500 Mikrometer. In einer weiteren Ausführungsform beträgt die Dicke der Klebstoffschicht etwa 50 Mikrometer bis etwa 100 Mikrometer. Vorzugsweise bildet die Klebstoffschicht eine im Wesentlichen durchgehende Schicht auf der Innenoberfläche der Trägerschicht, d. h. die Innenoberfläche der Trägerschicht ist im Wesentlichen vollflächig mit dem Haftklebstoff beschichtet. Die Innenoberfläche der Trägerschicht kann aber auch nur teilweise mit dem Haftklebstoff beschichtet sein, sodass z. B. höchstens 90%, höchstens 80%, höchstens 70%, höchstens 60%, höchstens 50% oder höchstens 40% der Innenoberfläche der Trägerschicht als Haftoberfläche ausgebildet sind.

Die Größe und Form der flexiblen Trägerschicht sind nicht sonderlich beschränkt. Als zweckmäßig hat sich z. B. ein runder bzw. ovaler Querschnitt der Trägerschicht erwiesen, wobei der Durchmesser der Trägerschicht vorzugsweise zwischen etwa 8 Zentimetern und etwa 13 Zentimetern liegt. Diese Ausführungen haben sich unter anderem für die Anbringung des Hygienepflasters in den Achselhöhlen als besonders geeignet erwiesen. Es versteht sich, dass Variationen der Trägerschicht in Bezug auf Form und Größe ohne Weiteres möglich sind, um das Hygienepflaster beispielsweise zweckspezifisch oder auch benutzerspezifisch anzupassen.

Auf der flexiblen Trägerschicht bzw. auf der Haftoberfläche ist abschnittsweise eine Saugauflage angeordnet, welche dazu ausgebildet ist, den von der menschlichen Haut austretenden Schweiß aufzunehmen. Der Begriff "abschnittsweise" ist so zu verstehen, dass die Saugauflage einen geringeren Durchmesser bzw. einen geringeren Umfang als die Trägerschicht aufweist und die Innenoberfläche der Trägerschicht bzw. die darauf angeordnete Klebstoffschicht daher nur teilweise überlagert. In bevorzugten Ausführungen sind höchstens 80%, höchstens 70%, höchstens 60%, höchstens 50%, höchstens 40%, höchstens 30%, höchstens 25% oder höchstens 20% der Innenoberfläche der Trägerschicht bzw. der darauf angeordneten Klebstoffschicht von der Sauauflage bedeckt. Auf diese Weise wird eine effiziente Schweißaufnahme bei gleichzeitig guter Haftwirkung des Pflasters erreicht. Die Saugauflage kann ebenso wie die Trägerschicht einen runden bzw. ovalen Querschnitt aufweisen, wobei der Durchmesser vorzugsweise zwischen 4 Zentimetern und 8 Zentimetern liegt. Selbstverständlich sind aber auch andere Querschnittsformen der Saugauflage möglich. Vorzugsweise ist die Saugauflage im Wesentlichen zentral auf der Trägerschicht bzw. der Haftoberfläche angeordnet.

Zwischen der flexiblen Trägerschicht und der Saugauflage bzw. zwischen der Haftoberfläche und der Saugauflage ist bei dem erfindungsgemäßen Hygienepflaster zumindest teilweise eine flüssigkeitsundurchlässige Sperrschicht angeordnet, welche dazu ausgebildet ist, einen Durchtritt des von der Saugauflage aufgenommenen Schweißes zu der flexiblen Trägerschicht bzw. zu der Haftoberfläche zu verhindern. Vorzugsweise sind die Saugauflage und die Trägerschicht bzw. die Haftoberfläche vollständig durch die flüssigkeitsundurchlässige Sperrschicht voneinander getrennt.

Die Erfinder haben erkannt, dass bei konventionell ausgebildeten Hygienepflastern der aufgenommene Schweiß durch die Saugauflage dringen kann, sodass die Trägerschicht bzw. die Haftoberfläche unterhalb der Saugauflage mit Flüssigkeit in Kontakt kommen. Es hat sich gezeigt, dass dieser Flüssigkeitskontakt ursächlich zu den bekannten Problemen herkömmlicher Hygienepflaster beiträgt. Zunächst führt durchtretender Schweiß in der Regel dazu, dass sich die Saugauflage von der Trägerschicht bzw. von der Haftoberfläche ablösen und insbesondere bei Bewegung zwischen der Trägerschicht und der Haut verrutschen kann. Dies beeinträchtigt den Tragekomfort und die Anwendungssicherheit des Hygienepflasters erheblich, da die Saugauflage in diesem Fall in der Regel nicht mehr optimal platziert ist. Darüber hinaus kann Schweiß, der aus der Saugauflage tritt, auch durch die Trägerschicht weiter nach außen dringen und auf diese Weise trotz des Pflasters zu Schweißflecken und Achselgeruch führen. Schließlich kann der Durchtritt größerer Flüssigkeitsmengen die teilweise oder vollständige Ablösung der Haftoberfläche von der Haut bewirken und dementsprechend zu einem völligen Funktionsversagen des Hygienepflasters führen.

Diese Probleme werden durch die flüssigkeitsundurchlässige Sperrschicht des erfindungsgemäßen Hygienepflasters gelöst. Damit bleibt eine zuverlässige Funktion des Hygienepflasters gerade auch bei erhöhtem Schweißaustritt und längeren Anwendungszeiten gewährleistet. Gleichzeitig ist es mithilfe der Sperrschicht möglich, die Kapazität der Saugauflage zur Schweißaufnahme auf ein für den jeweiligen Anwendungsfall erforderliches Mindestmaß zu beschränken, sodass die Saugauflage verhältnismäßig dünner als bei herkömmlichen Hygienepflastern ausgebildet sein kann. Dies führt insbesondere in dem für einen Materialauftrag oft empfindlichen Achselbereich zu einer Verbesserung des Tragekomforts.

Die Ausführung der Sperrschicht ist nicht sonderlich beschränkt, sofern die erfindungsgemäße Wirkung einer Flüssigkeitssperre zwischen der Saugauflage und der Trägerschicht bzw. der Haftoberfläche realisiert wird. Die Sperrschicht kann einen flüssigkeitsundurchlässigen Film bzw. eine flüssigkeitsundurchlässige Membran oder Folie aus z. B. Polyethylen, Polypropylen und/oder Silikon umfassen bzw. daraus bestehen. Hydrophobe Materialien bzw. hydrophobe Beschichtungen sind bevorzugt, da diese auch antibakteriell wirken und somit zusätzlich die Geruchsbildung hemmen können. Die Schichtdicke der Sperrschicht kann 1 bis 100 Mikrometer betragen, wobei Schichtdicken von etwa 5 Mikrometer bis etwa 50 Mikrometer und insbesondere von etwa 10 Mikrometer bis etwa 25 Mikrometer bevorzugt sind. Weitere geeignete Ausführungsformen für die Sperrschicht kann der Fachmann ohne Weiteres auf der Grundlage dieser Offenbarung bestimmen.

Die Saugauflage kann einteilig oder mehrteilig aufgebaut sein. In bevorzugten Ausführungsformen umfasst die Saugauflage mindestens zwei, mindestens drei oder auch mehr unterschiedliche Schichten bzw. unterschiedliche Vliesschichten, wobei "Vliesschicht" im Sinne der vorliegenden Erfindung als Sammelbegriff für ein dreidimensional strukturiertes Faserflächengebilde verwendet wird. Die verschiedenen Vliesschichten können sich insbesondere strukturell und/oder funktionell unterscheiden und zum Beispiel unterschiedliche Fasermaterialien, unterschiedliche Faserorientierungen, unterschiedlichen Faseraufbau, unterschiedliche Faserlängen und/oder unterschiedliche Flächengewichte aufweisen.

Die Saugauflage weist ein Speichervlies auf, welches ein fluidabsorbierendes Mittel enthält, wobei das fluidabsorbierende Mittel ein Superabsorber in Granulatform ist, der in das Speichervlies eingearbeitet ist. Bevorzugt sind solche fluidabsorbierende Mittel, die zusammen mit Flüssigkeit ein Hydrogel ausbilden. Dabei handelt es sich insbesondere um hydrophile Polymere, welche in der Lage sind, große Mengen wässriger Flüssigkeit aufzunehmen ohne sich in der Flüssigkeit zu lösen, auch als "Superabsorber" bezeichnet. Dies hat zum einen den Vorteil, dass die Saugauflage wesentlich dünner als bei der Verwendung herkömmlicher Absorptionsmaterialien ausgebildet sein kann, wofür der Superabsorber auch im Vlies bzw. in das Gewebe- oder Fasermaterial eingearbeitet sein kann. Ein weiterer wesentlicher Vorteil ist, dass Bindung der Schweißflüssigkeit in Form eines Hydrogels das Bakterienwachstum hemmt und somit einer Geruchsbildung effizient entgegenwirkt.

Geeignete fluidabsorbierende Mittel können beispielsweise Gelatine, Polysaccharide wie z. B. Cellulose oder Cellulosederivate, Acrylamidpolymere, thermoplastische Polyurethane, vernetzte Acrylate bzw. Methacrylate, Polymere oder Copolymere von N-Vinylpyrrolidon oder Polymere oder Copolymere von Acrylsäure bzw. deren Salzen enthalten oder daraus bestehen. Auch beliebige Kombinationen dieser Substanzen sind möglich. Besonders bevorzugt sind vernetzte Acrylate und Methacrylate sowie Derivate davon, beispielsweise Polyalkylenglykolacrylat bzw. Polyalkylenglykolmethacrylat und deren substituierte Derivate, Poly(2-Acrylamido-2-methylpropansulfonat) (PolyAMPS) und dessen Salze oder Poly(2-hydroxyethylmethacrylat) und dessen Salze. In einer bevorzugten Ausführungsform weist das Speichervlies eine Kombination von Cellulose und Natriumpolyacrylat auf. Das fluidabsorbierende Mittel kann z. B. in Form von Partikeln bzw. eines Pulvers, eines Granulats, Fasern oder dergleichen in dem Speichervlies enthalten sein.

Die Saugauflage kann weiterhin ein Innenvlies aufweisen, welches bei Verwendung des Hygienepflasters auf der Haut aufliegt. Das Innenvlies ist so ausgebildet, dass der Schweiß von der Haut aufgenommen und in die Saugauflage abgeleitet wird. Auf diese Weise bleibt die Haut unter dem Pflaster weitgehend trocken, wodurch einer Geruchsbildung wirksam vorgebeugt und ein angenehmes Tragegefühl erreicht wird. Ein geeignetes Innenvlies kann z. B. Polyethylenfasern enthalten oder aus solchen bestehen. Solche Vliese weisen eine vorteilhafte hohe Saugwirkung auf und vermitteln gleichzeitig auf der Haut ein angenehmes Tragegefühl.

Die Saugauflage kann außerdem ein Verteilervlies aufweisen, welches dazu ausgebildet ist, den Schweiß in der Saugauflage zu verteilen. So, wie der Begriff hier verwendet wird, bedeutet "verteilen", dass der Schweiß teilweise oder überwiegend in einer lateralen Ausdehnungsrichtung, d. h. in Richtung der Verteilervliesebene bzw. über den Querschnitt der Saugauflage geleitet wird, wohingegen das Innenvlies den Schweiß insbesondere in einer axialen Ausdehnungsrichtung transportiert, d. h. entlang der Höhe bzw. Dicke der Saugauflage. In bevorzugten Ausführungsformen sind sowohl das Verteilervlies als auch das Innenvlies vorhanden, wobei das Verteilervlies auf einer von der Haut abgewandten Innenseite des Innenvlieses angeordnet ist. Vorzugsweise grenzt das Verteilervlies unmittelbar an das Innenvlies an bzw. steht mit diesem im Kontakt, sodass der über das Innenvlies ins Innere der Saugauflage geleitete Schweiß mithilfe des Verteilervlieses unverzüglich in der Saugauflage verteilt wird. Auf diese Weise lässt sich die Kapazität der Saugauflage zur Schweißaufnahme sehr gleichmäßig und effizient nutzen. Dadurch wird die erforderliche Materialmenge in der Saugauflage gegenüber herkömmlichen Hygienepflastern reduziert, was zum einen eine Kostenersparnis und zum anderen einen erhöhten Tragekomfort bewirkt. Das Verteilervlies kann z. B. Polyethylenfasern, Polypropylenfasern, Cellulosefasern bzw. Fasern eines Cellulosederivats oder beliebige Kombinationen davon enthalten oder aus diesen bestehen.

Eine besonders günstige Zusammenwirkung von Innenvlies und Verteilervlies wird erreicht, wenn das Innenvlies zumindest teilweise eine größere Porengröße, d. h. insbesondere einen größeren mittleren Porendurchmesser, als das Verteilervlies aufweist. Die größere Porengröße steigert die Benetzbarkeit bzw. die Permeabilität des Innenvlieses und begünstigt einen raschen Transport der Schweißflüssigkeit ins Innere der Saugauflage, wo die geringere Porengröße des Verteilervlieses für eine entsprechende Ausbreitung und flächige Verteilung der Schweißflüssigkeit innerhalb der Saugauflage bewirkt. Alternativ oder zusätzlich kann das Innenvlies auch zumindest teilweise eine andere Faserorientierung als das Verteilervlies aufweisen, um dieselben Effekte zu erzielen. Beispielsweise kann das Innenvlies im Vergleich zum Verteilervlies einen größeren Teil an Fasern enthalten, welche im Wesentlichen senkrecht zur Vliesebene ausgerichtet sind. Es ist auch möglich, dass das Verteilervlies ein höheres Flächengewicht als das Innenvlies aufweist, wodurch die laterale Ausbreitung der über das Innenvlies ankommenden Schweißflüssigkeit in dem Verteilervlies zusätzlich gefördert wird. Auch Kombinationen der vorgenannten Eigenschaften sind möglich.

Geeignete Methoden zur Herstellung der verschiedenen Vliesschichten wie z. B. aerodynamische (engl. "airlaid") Schmelz-Blas-Verfahren (engl. "Meltblown"-Verfahren) und Vliesbildungsverfahren auf Spinnfaserbasis mit ggf. anschließendem mechanischen, chemischen oder thermischen Verfestigungsverfahren sind dem Fachmann bekannt. Es ist möglich, die Saugauflage dabei einstückig oder mehrstückig auszubilden. In einer bevorzugten einstückigen Ausführung bilden Speichervlies, Verteilervlies und/oder Innenvlies verschiedene Bereiche innerhalb desselben zusammenhängenden Fasergebildes. Es ist auch möglich, dass z. B. Speichervlies und Verteilervlies bzw. Verteilervlies und Innenvlies zumindest teilweise einen gemeinsamen Bereich innerhalb des Fasergebildes ausformen, d. h. derselbe Vliesbereich kann z. B. sowohl die Funktion des Speichervlieses als auch die Funktion des Verteilervlieses in sich vereinen. Eine mehrstückige Ausführung sieht vor, dass Speichervlies, Verteilervlies und/oder Innenvlies jeweils separat hergestellt werden und erst anschließend in Form der Saugauflage zusammengeführt sowie ggf. miteinander verbunden werden, z. B. durch thermisches Verschweißen. Diesbezüglich wird auch auf die nachfolgenden Ausführungsbeispiele verwiesen.

Die Saugauflage kann mit Wirk- und/oder Duftstoffen versetzt sein, um Geruchsbildung zu reduzieren bzw. zu verhindern oder zu neutralisieren. Insbesondere kann die Saugauflage Silber (z. B. Silberfäden) bzw. Silberionen enthalten, die mit Proteinen von Bakterienzellen Komplexe bilden und dadurch bakteriostatisch bzw. bakteriozid wirken. Auf diese Weise wird selbst bei langen Anwendungszeiten des erfindungsgemäßen Hygienepflasters einer Entwicklung von Schweißgeruch effektiv vorgebeugt. Möglich ist auch, dass der Superabsorber in das Vliesgewebe der Speicherschicht bzw. in das Fasermaterial bereits eingearbeitet ist und dieser Schicht gleichzeitig eine oder mehrere Substanzen ausgewählt aus Aluminium, Chlorid, Aluminiumchlorohydrat (Aluminiumhydroxychlorid), Glycerin und Propylenglykol beigefügt sind, um den Schweißgeruch zu vermindern. Da diese Substanzen in der Speicherschicht bzw. dem Speichervlies verteilt vorliegen, haben sie durch den erfindungsgemäßen Aufbau der Saugauflage auch keinen direkten Hautkontakt, neutralisieren also in vorteilhafter Weise den Schweißgeruch ohne Hautreaktionen hervorzurufen.

In einer bevorzugten Ausführung weist die Saugauflage einen oder mehrere Einschnitte auf, welche sich von einer umlaufenden Außenkante der Saugauflage vorzugsweise radial verlaufend in die Saugauflage erstrecken. Dies hat den Vorteil, dass die Saugauflage besser an die Körperkonturen anpassbar ist und ein erhöhter Tragekomfort erreicht wird. Zudem reduzieren die Einschnitte das Walken des Pflasters durch Körperbewegung und wirken dadurch einer Materialermüdung und Ablösung des Pflasters auch bei stärkerer mechanischer Belastung entgegen. Vorzugsweise weist die Saugauflage mindestens zwei gegenüberliegende Einschnitte auf. Besonders bevorzugt weist die Auflage mindestens drei oder mindestens vier Einschnitte auf, die gleichmäßig über den Umfang der Saugauflage verteilt sind. Die Länge der Einschnitte beträgt vorzugsweise jeweils unabhängig voneinander mindestens 5% und höchstens 50%, vorzugsweise zwischen 10% und 30% des Durchmessers der Saugauflage.

Schließlich kann das Hygienepflaster eine Schutz- bzw. Abziehschicht aufweisen, welche entfernbar auf der Haftoberfläche der Trägerschicht angeordnet ist. Auf diese Weise wird verhindert, dass die Haftoberfläche verschmutzt oder vor Gebrauch des Pflasters auf einer anderen als der dafür vorgesehenen Körperoberfläche haftet. In bevorzugten Ausführungen entsprechen Größe und Form der Schutz- bzw. Abziehschicht im Wesentlichen jeweils derjenigen der Trägerschicht. Dadurch ist es möglich, die Saugauflage zwischen der Trägerschicht bzw. der Haftoberfläche und der Schutzschicht anzuordnen bzw. einzuschließen. Dies hat den Vorteil, dass die Saugauflage vor der Verwendung des Pflasters nicht mit Schmutz oder Feuchtigkeit in Berührung kommen kann, die ggf. die Funktion des Hygienepflasters beeinträchtigen könnten. Die Schutz- bzw. Abziehschicht kann z. B. als Folie aus Polyethylen und/oder Polypropylen ausgebildet sein, wobei die Folie auf derjenigen Oberfläche, welche die Haftoberfläche kontaktiert, eine Beschichtung mit Silikon, einer Silikonzusammensetzung oder dergleichen aufweisen kann, um die Entfernung der Folie von der Haftoberfläche zu erleichtern.

Die Erfindung wird nachfolgend anhand der beigefügten Figuren näher erläutert. Diese stellen lediglich schematische Prinzipdarstellungen dar und sind nur beispielhaft zu verstehen. Keinesfalls soll die Erfindung auf die gezeigten Figuren beschränkt sein. Um die Erfindung besser verdeutlichen zu können, sind die Merkmale in den Figuren nicht maßstabsgetreu wiedergegeben. Es zeigen:
- Fig. 1: eine perspektivische Ansicht eines erfindungsgemäßen Hygienepflasters;
- Fig. 2: eine Schnittdarstellung des Hygienepflasters aus Fig. 1;
- Fig. 3: eine Schnittdarstellung einer weiteren Ausführungsform des Hygienepflasters aus Fig 1.

Gleiche Bezugszeichen in den Figuren deuten auf gleiche oder analoge Elemente hin.

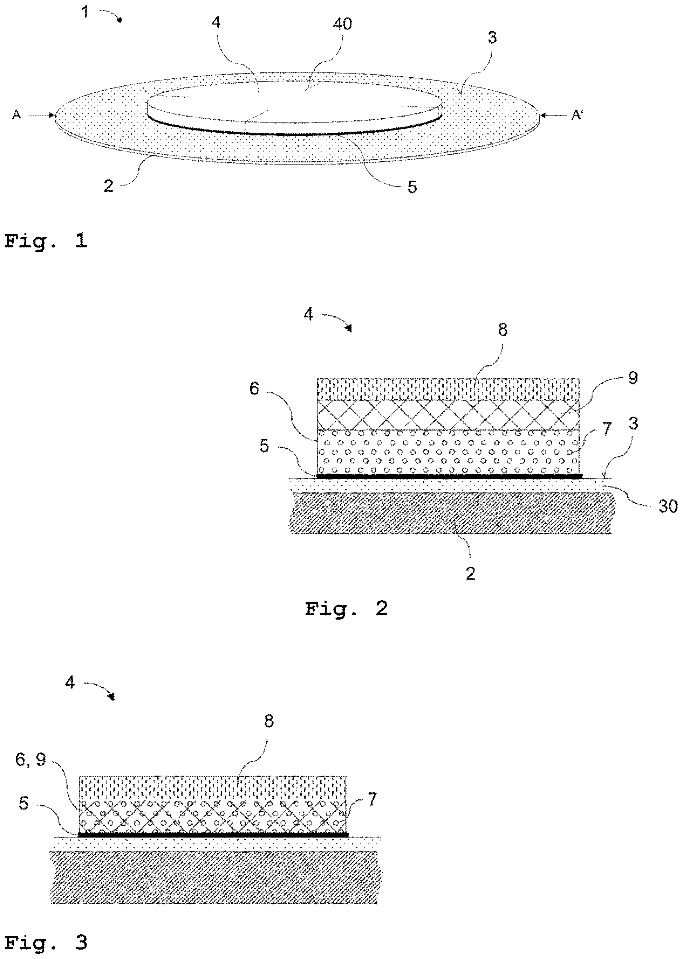

Der generelle Aufbau eines erfindungsgemäßen Hygienepflasters 1 ist in einer stark vereinfachten perspektivischen Ansicht in Figur 1 gezeigt. Das Hygienepflaster 1 weist eine flexible Trägerschicht 2 auf, deren Oberseite als Haftoberfläche 3 ausgebildet ist. In einem zentralen Abschnitt auf der Trägerschicht 2 bzw. auf der Haftoberfläche 3 ist eine Saugauflage 4 angeordnet. Die Saugauflage 4 ist von einem geringeren Durchmesser als die Trägerschicht 2, sodass deren Haftoberfläche 3 im Peripheriebereich der Trägerschicht 2 freiliegt. Auf diese Weise lässt sich das Hygienepflaster 1 im Anwendungsfall über die Haftoberfläche 3 auf der Haut fixieren. Die Saugauflage 4 und die Haftoberfläche 3 sind durch die flüssigkeitsundurchlässige Sperrschicht 5 voneinander getrennt. Dies verhindert, dass Schweiß, der von der Saugauflage 4 aufgenommen wird, zu der Trägerschicht 2 bzw. der Haftoberfläche 3 durchdringen kann. Mithilfe der vier optionalen, über den Umfang der Saugauflage 4 gleichmäßig verteilten Einschnitte 40 wird im Anwendungsfall eine besonders gute Anpassbarkeit der Saugauflage 4 an die Körperkonturen wie z. B. die Achselhöhle erreicht und die Walkarbeit des Pflasters unter Bewegungsbelastung herabgesetzt.

In Figur 2 ist ein bevorzugter Aufbau des Hygienepflasters 1 anhand einer Schnittdarstellung entlang der in Figur 1 gezeigten Schnittachse A-A` näher dargestellt. Die Trägerschicht 2 kann zum Beispiel ein querelastisches Polyestervlies sein. Die Elastizität ist von Vorteil, um bei Anwendung einen permanent vollflächigen Hautkontakt des Hygienepflasters 1 auch auf stark bewegten Körperstellen wie z. B. den Achselhöhlen zu gewährleisten. Die bei Anwendung des Hygienepflasters 1 zur Haut weisende Oberfläche der Trägerschicht 2 ist mit einer Haftklebstoffschicht 30 beschichtet und bildet auf diese Weise die Haftoberfläche 3 aus. Als Haftklebstoff hat sich zum Beispiel eine Beschichtung mit einer selbstklebenden Zinkoxid-Kautschuk-Klebemasse oder Polyacrylat-Klebemasse bewährt, welche zuverlässige Hafteigenschaften auf der einen Seite und gute Hautverträglichkeit auf der anderen Seite besonders vorteilhaft miteinander vereinen. Zwischen der Haftoberfläche 3 und der Saugauflage 4 ist die Sperrschicht 5 in Form einer flüssigkeitsundurchlässigen Polyethylen- bzw. Polypropylenmembran angeordnet. Es ist auch möglich, dass die Sperrschicht 5 direkt auf der Trägerschicht 2 angeordnet ist, wenn beispielsweise lediglich der umlaufende äußere Randbereich der Trägerschicht 2 als Haftoberfläche 3 ausgebildet ist und dementsprechend keine Klebstoffschicht 30 in dem Abschnitt unterhalb der Saugauflage 4 vorliegt (hier nicht dargestellt).

Figur 2 zeigt weiterhin, dass die Saugauflage 4 aus drei unterschiedlichen Schichten bzw. Lagen aufgebaut sein kann. Die unterste Schicht wird hier von einem Speichervlies 6 gebildet, in das ein fluidabsorbierendes Mittel 7 integriert ist. Geeignet ist z. B. ein Cellulosevlies mit darin eingebrachten superabsorbierenden Partikeln aus Natriumpolyacrylat. Die äußerste Schicht, welche bei Anwendung des Hygienepflasters 1 die Haut kontaktiert, besteht aus einem Innenvlies 8, das den von der Haut austretenden Schweiß schnell aufsaugt und in das Innere der Saugauflage 4 transportiert. Großporige Polyethylen-Vliesstoffe haben sich hierfür als besonders zweckmäßig erwiesen. Zwischen dem Speichervlies 6 und dem Innenvlies 8 befindet sich das Verteilervlies 9, welches dazu ausgelegt ist, die von dem Innenvlies 8 ankommende Schweißflüssigkeit über den Querschnitt der Saugauflage 4 auszubreiten, um die Aufnahmekapazität der Speicherschicht 6 möglichst gleichmäßig und effizient auszunutzen. Eine gute Verteilungswirkung lässt sich z. B. mithilfe von Vliesstoffen erreichen, welche Mischungen von Polyethylen-, Polypropylen- und/oder Cellulosefasern enthalten. Die verschiedenen Vliesschichten 6, 8, 9 können zunächst separat z. B. im Airlaid-Verfahren hergestellt werden. Anschließend werden die Vliesschichten 6, 8, 9 übereinander angeordnet und z. B. durch thermisches Verschweißen miteinander verbunden, um die Saugauflage 4 auszubilden

Schließlich zeigt die Figur 3 eine alternative Ausgestaltung der Saugauflage 4. Im Unterschied zu dem in Figur 2 gezeigten Beispiel liegt die Saugauflage 4 hier als einstückiges Vlies vor, in dem verschiedene Vliesschichten enthalten bzw. kombiniert sind. Der obere, zur Auflage auf die Haut vorgesehene Bereich des Vlieses ist als Innenvlies 8 ausgebildet. Das Innenvlies 8 geht in einen unteren Bereich über, in dem Speichervlies 6 und Verteilervlies 9 miteinander kombiniert vorliegen. Ein ensprechendes Vlies ist z. B. von der Fa. Glatfelter Falkenhagen GmbH (Pritzwalk, Deutschland) erhältlich. Auf diese Weise lassen sich erfindungsgemäß überraschend dünne Hygienepflaster 1 bereitstellen, die einen erhöhten Tragekomfort bei gleichzeitig hoher Schutzwirkung bieten.

### Bezugszeichenliste

- 1: Hygienepflaster
- 2: Trägerschicht
- 3: Haftoberfläche
- 4: Saugauflage
- 5: Sperrschicht
- 6: Speichervlies
- 7: fluidabsorbierendes Mittel
- 8: Innenvlies
- 9: Verteilervlies
- 30: Klebstoffschicht
- 40: Einschnitt

## Patentansprüche

1. Eine Verwendung eines Hygienepflaster (1) zur Aufnahme von Schweiß menschlicher Haut, umfassend
eine flexible Trägerschicht (2) mit einer an der menschlichen Haut fixierbaren Haftoberfläche (3) und
eine auf der Haftoberfläche (3) abschnittsweise angeordneten Saugauflage (4), welche dazu ausgebildet ist, den von der menschlichen Haut austretenden Schweiß aufzunehmen,
wobei zwischen der flexiblen Trägerschicht (2) und der Saugauflage (4) zumindest teilweise eine flüssigkeitsundurchlässige Sperrschicht (5) angeordnet ist, welche dazu ausgebildet ist, einen Durchtritt des von der Saugauflage (4) aufgenommenen Schweißes zu der flexiblen Trägerschicht (2) zu verhindern,
wobei die Saugauflage (4) ein Speichervlies (6) aufweist, welches ein fluidabsorbierendes Mittel (7) enthält, wobei das fluidabsorbierende Mittel ein Superabsorber in Granulatform ist, der in das Speichervlies eingearbeitet ist,
zur Aufbringung auf der Haut in den Achselhöhlen.

2. Die Verwendung des Hygienepflasters (1) nach Anspruch 1, wobei die Sperrschicht (5) einen flüssigkeitsundurchlässigen Film oder eine flüssigkeitsundurchlässige Membran oder Folie aus Polyethylen und/oder Polypropylen umfasst oder daraus besteht.

3. Die Verwendung des Hygienepflasters (1) nach Anspruch 1 oder 2, wobei die Saugauflage (4) mindestens zwei oder mindestens drei unterschiedliche Vliesschichten (6, 8, 9) umfasst.

4. Die Verwendung des Hygienepflasters (1) nach Anspruch 1, wobei das fluidabsorbierende Mittel (7) ein Polymer oder Copolymer von Acrylsäure, einem Acrylsäuresalz und/oder Acrylamid, Cellulose oder ein Cellulosederivat oder beliebige Kombinationen davon enthält oder daraus besteht.

5. Die Verwendung des Hygienepflasters (1) nach einem der Ansprüche 1 bis 4, wobei die Saugauflage (4) ein Innenvlies (8) aufweist, welches bei Verwendung des Hygienepflasters (1) auf der Haut aufliegt und dazu ausgebildet ist, den Schweiß von der Haut aufzunehmen und in die Saugauflage (4) zu leiten.

6. Die Verwendung des Hygienepflasters (1) nach Anspruch 5, wobei das Innenvlies (8) Polyethylenfasern enthält oder aus diesen besteht.

7. Die Verwendung des Hygienepflasters (1) nach einem der Ansprüche 1 bis 6, wobei die Saugauflage (4) mindestens ein Verteilervlies (9) aufweist, welches dazu ausgebildet ist, den Schweiß in der Saugauflage (4) zu verteilen.

8. Die Verwendung des Hygienepflasters (1) nach Anspruch 7, soweit zurückbezogen auf einen der Ansprüche 5 oder 6, wobei das Verteilervlies (9) auf einer von der Haut abgewandten Innenseite des Innenvlieses (8) angeordnet ist und/oder unmittelbar an das Innenvlies (8) angrenzt.

9. Die Verwendung des Hygienepflasters (1) nach einem der Ansprüche 7 bis 8, wobei das Verteilervlies (9) Polyethylenfasern, Polypropylenfasern, Cellulosefasern bzw. Fasern eines Cellulosederivates oder beliebige Kombinationen davon enthält oder aus diesen besteht.

10. Die Verwendung des Hygienepflasters (1) nach einem der Ansprüche 7 bis 9, soweit zurückbezogen auf einen der Ansprüche 5 oder 6, wobei das Innenvlies (8) zumindest teilweise eine größere Porengröße und/oder zumindest teilweise eine andere Faserorientierung als das Verteilervlies (9) aufweist.

11. Die Verwendung des Hygienepflasters (1) nach einem der Ansprüche 1 bis 10, wobei die Saugauflage (4) Silber und/oder Silberionen enthält.

12. Die Verwendung des Hygienepflasters (1) nach einem der Ansprüche 1 bis 11, wobei die Saugauflage (4) einen oder mehrere Einschnitte (40) aufweist, welche sich von einer Außenkante der Saugauflage (4) in die Saugauflage (4) erstrecken.

13. Die Verwendung des Hygienepflasters (1) nach Anspruch 12, wobei der oder die Einschnitte (40) bezogen auf die Saugauflage (4) radial verlaufen.

14. Die Verwendung des Hygienepflasters (1) nach einem der Ansprüche 1 bis 13, wobei die flexible Trägerschicht (2) ein querelastisches Polyestervlies umfasst oder daraus besteht.

15. Die Verwendung des Hygienepflasters (1) nach einem der Ansprüche 1 bis 14, wobei die flexible Trägerschicht (2) einen runden oder ovalen Querschnitt mit einem Durchmesser von 8 Zentimetern bis 13 Zentimetern aufweist,
und/oder die Saugauflage (4) einen runden oder ovalen Querschnitt mit einem Durchmesser von 4 Zentimetern bis 8 Zentimetern aufweist.

## Claims

1. A use of a sanitary patch (1) for absorbing sweat from human skin, comprising
a flexible carrier layer (2) with an adhesive surface (3) which can be fixed to the human skin, and
an absorbent pad (4) arranged in a portion on the adhesive surface (3), which is configured to absorb the sweat exuding from the human skin,
wherein a liquid-impermeable barrier layer (5) is arranged at least partially between the flexible carrier layer (2) and the absorbent pad (4), which is configured to prevent the sweat absorbed by the absorbent pad (4) from passing through to the flexible carrier layer (2),
wherein the absorbent pad (4) comprises a storage fleece (6) which contains a fluid-absorbing agent (7), wherein the fluid-absorbing agent is a superabsorber in granular form which is embedded in the storage fleece,
for application to the skin in the armpits.

2. The use of the sanitary patch (1) according to claim 1, wherein the barrier layer (5) comprises or consists of a liquid-impermeable film or a liquid-impermeable membrane or sheet of polyethylene and/or polypropylene.

3. The use of the sanitary patch (1) according to claim 1 or 2, wherein the absorbent pad (4) comprises at least two or at least three different nonwoven layers (6, 8, 9).

4. The use of the sanitary patch (1) according to claim 1, wherein the fluid-absorbing agent (7) comprises or consists of a polymer or copolymer of acrylic acid, an acrylic acid salt and/or acrylamide, cellulose or a cellulose derivative or any combinations thereof.

5. The use of the sanitary patch (1) according to one of claims 1 to 4, wherein the absorbent pad (4) comprises an inner fleece (8) which rests on the skin when the sanitary patch (1) is used and is configured to absorb sweat from the skin and guide it into the absorbent pad (4).

6. The use of the sanitary patch (1) according to claim 5, wherein the inner fleece (8) comprises or consists of polyethylene fibers.

7. The use of the sanitary patch (1) according to any one of claims 1 to 6, wherein the absorbent pad (4) comprises at least one distribution fleece (9) configured to distribute the sweat in the absorbent pad (4).

8. The use of the sanitary patch (1) according to claim 7, as far as depending on one of claims 5 or 6, wherein the distribution fleece (9) is arranged on an inner side of the inner fleece (8) facing away from the skin and/or is directly adjacent to the inner fleece (8).

9. The use of the sanitary patch (1) according to any one of claims 7 to 8, wherein the distribution fleece (9) comprises or consists of polyethylene fibers, polypropylene fibers, cellulose fibers or fibers of a cellulose derivative or any combination thereof.

10. The use of the sanitary patch (1) according to any one of claims 7 to 9, as far as related back to any one of claims 5 or 6, wherein the inner fleece (8) comprises at least partially a larger pore size and/or at least partially a different fiber orientation than the distribution fleece (9).

11. The use of the sanitary patch (1) according to any one of claims 1 to 10, wherein the absorbent pad (4) contains silver and/or silver ions.

12. The use of the sanitary patch (1) according to any one of claims 1 to 11, wherein the absorbent pad (4) comprises one or more incisions (40) extending from an outer edge of the absorbent pad (4) into the absorbent pad (4).

13. The use of the sanitary patch (1) according to claim 12, wherein the incision or the incisions (40) extend radially with respect to the absorbent pad (4).

14. The use of the sanitary patch (1) according to any one of claims 1 to 13, wherein the flexible carrier layer (2) comprises or consists of a transversely elastic polyester nonwoven.

15. The use of the sanitary patch (1) according to any one of claims 1 to 14, wherein the flexible carrier layer (2) comprises a round or oval cross-section with a diameter of 8 centimeters to 13 centimeters,
and/or wherein the absorbent pad (4) comprises a round or oval cross-section with a diameter of 4 centimeters to 8 centimeters.

## Revendications

1. Utilisation d'un timbre hygiénique (1) destiné à s'imbiber de la sueur d'une peau humaine, comprenant
une couche de support (2) flexible comportant une surface adhésive (3) pouvant être fixée sur la peau humaine et
une garniture d'aspiration (4) disposée dans certaines zones sur la surface adhésive (3), laquelle garniture d'aspiration est conçue pour s'imbiber de la sueur émanant de la peau humaine,
dans laquelle, entre la couche de support (2) flexible et la garniture d'aspiration (4), est disposée au moins partiellement une couche barrière (5) imperméable aux liquides, laquelle couche barrière est conçue pour empêcher un passage de la sueur dont la garniture d'aspiration (4) s'est imbibée vers la couche de support (2) flexible,
dans laquelle la garniture d'aspiration (4) présente un non-tissé de stockage (6) qui contient un moyen absorbant les fluides (7), dans laquelle le moyen absorbant les fluides est un superabsorbant sous forme de granulés, lequel superabsorbant est incorporé dans le non-tissé de stockage,
destiné à être appliqué sur la peau au niveau des aisselles.

2. Utilisation du timbre hygiénique (1) selon la revendication 1, dans laquelle la couche barrière (5) comprend ou est constituée d'un film imperméable aux liquides ou d'une membrane ou feuille en polyéthylène et/ou en polypropylène, imperméable aux liquides.

3. Utilisation du timbre hygiénique (1) selon la revendication 1 ou 2, dans laquelle la garniture d'aspiration (4) comprend au moins deux ou au moins trois couches de non-tissé (6, 8, 9) différentes.

4. Utilisation du timbre hygiénique (1) selon la revendication 1, dans laquelle le moyen absorbant les fluides (7) contient ou est constitué d'un polymère ou copolymère d'acide acrylique, sel d'acide acrylique et/ou acrylamide, cellulose ou dérivé de cellulose, ou de toute combinaison de ceux-ci.

5. Utilisation du timbre hygiénique (1) selon l'une des revendications 1 à 4, dans laquelle la garniture d'aspiration (4) présente un non-tissé intérieur (8) qui, lors de l'utilisation du timbre hygiénique (1), est en contact avec la peau et est conçu pour s'imbiber de la sueur de la peau et la diriger dans la garniture d'aspiration (4).

6. Utilisation du timbre hygiénique (1) selon la revendication 5, dans laquelle le non-tissé intérieur (8) contient ou est constitué de fibres de polyéthylène.

7. Utilisation du timbre hygiénique (1) selon l'une des revendications 1 à 6, dans laquelle la garniture d'aspiration (4) présente au moins un non-tissé de répartition (9) qui est conçu pour répartir la sueur dans la garniture d'aspiration (4).

8. Utilisation du timbre hygiénique (1) selon la revendication 7, dans la mesure où elle se rapporte à l'une des revendications 5 ou 6, dans laquelle le non-tissé de répartition (9) est disposé sur une face intérieure, opposée à la peau, du non-tissé intérieur (8) et/ou est directement adjacent au non-tissé intérieur (8).

9. Utilisation du timbre hygiénique (1) selon l'une des revendications 7 à 8, dans laquelle le non-tissé de répartition (9) contient ou est constitué de fibres de polyéthylène, fibres de polypropylène, fibres de cellulose ou fibres d'un dérivé de cellulose, ou de toute combinaison de celles-ci.

10. Utilisation du timbre hygiénique (1) selon l'une des revendications 7 à 9, dans la mesure où elle se rapporte à l'une des revendications 5 ou 6, dans laquelle le non-tissé intérieur (8) présente au moins partiellement une taille de pores plus grande que celle du non-tissé de répartition et/ou au moins partiellement une orientation de fibres différente de celle du non-tissé de répartition (9).

11. Utilisation du timbre hygiénique (1) selon l'une des revendications 1 à 10, dans laquelle la garniture d'aspiration (4) contient de l'argent et/ou des ions argent.

12. Utilisation du timbre hygiénique (1) selon l'une des revendications 1 à 11, dans laquelle la garniture d'aspiration (4) présente une ou plusieurs entailles (40) qui s'étendent dans la garniture d'aspiration (4) à partir d'un bord extérieur de la garniture d'aspiration (4).

13. Utilisation du timbre hygiénique (1) selon la revendication 12, dans laquelle l'entaille ou les entailles (40) évoluent radialement par rapport à la garniture d'aspiration (4).

14. Utilisation du timbre hygiénique (1) selon l'une des revendications 1 à 13, dans laquelle la couche de support (2) flexible comprend ou est constituée d'un non-tissé de polyester à élasticité transversale.

15. Utilisation du timbre hygiénique (1) selon l'une des revendications 1 à 14, dans laquelle la couche de support (2) flexible présente une section transversale ronde ou ovale d'un diamètre allant de 8 centimètres à 13 centimètres,
et/ou la garniture d'aspiration (4) présente une section transversale ronde ou ovale d'un diamètre allant de 4 centimètres à 8 centimètres.
